# EUROPEAN PATENT APPLICATION

(11) **EP 4 600 888 A1**
(43) Date of publication of application: **13.08.2025**
(21) Application number: 23888993.5
(22) Date of filing: 26.10.2023
(51) Int. Cl.: G06Q 30/02, G06N 20/00, G10L 25/51, G10L 13/02, G06F 40/35, G06F 18/24, H04M 3/493, G10L 15/08

(54) **METHOD FOR PERFORMING CUSTOMIZED CONSULTATION FOR EACH USER TYPE ON BASIS OF ARTIFICIAL INTELLIGENCE**

(30) Priority: 08.11.2022 KR 20220147443
(71) Applicant: Moon, Manki, Yangju-si Gyeonggi-do 11476 (KR)
(72) Inventor: Moon, Manki, Yangju-si Gyeonggi-do 11476 (KR)
(74) Representative: ABG Intellectual Property Law, S.L.
(86) International application number: PCT/KR2023/016772
(87) International publication number: WO 2024/101731

(57) **Abstract**

A method for controlling an electronic device according to an embodiment of the present invention comprises: a consultation step in which the electronic device provides a voice of a digital human(AI agent) including an image in which the digital human is visualized and at least one query, and obtains a user's response to the query; a measurement step in which the electronic device obtains measurement values for five measurement items including the user's disposition, virtue, personality, cognitive faculty, and personal environments according to the response; and a classification step in which the electronic device identifies, on the basis of the obtained measurement values, the user's type associated with tolerance to digital addiction.

## Description

### [TECHNICAL FIELD]

The present disclosure relates to a method for classifying user types and performing consultation for each user type on the basis of an artificial intelligence model.

### [BACKGROUND ART]

The problems occurring in previous research on behavioral addiction can be largely divided into two categories. The first is the conceptualization of behavioral addiction without distinguishing between online and offline, and the second is the problem of establishing diagnosis and clinical intervention strategies for behavioral addiction based on diagnostic criteria derived from existing substance (drug) addiction.

Due to a difference in self-efficacy between the virtual and real worlds, research on behavioral addiction is somewhat confusing, as it views behavioral addiction without distinguishing between offline and online. This phenomenon has been attributed to the lack of research on diagnostic criteria and components of offline and online behavioral addiction, which has led to unclear conceptualization.

Substance addiction is directly affected by the frequency and amount of exposure to a mediator (drugs, etc.), while behavioral addiction is significantly affected by the person's behavior and psychological characteristics, as well as socio-environmental factors. As a result, depending on the characteristics of the person, there are types that are vulnerable to addiction and types that are not, even for the same exposure time. Therefore, it is necessary to avoid relying on existing diagnostic criteria derived from drug addiction, and to develop diagnostic and response strategies for behavioral addictions that are tailored to the characteristics of the individual.

### [DETAILED DESCRIPTION OF INVENTION]

### [TECHNICAL PROBLEMS]

An embodiment of the present disclosure is provided to diagnose behavioral addiction and establish a corresponding strategy, not by using conventional diagnostic criteria derived from drug addiction, but by establishing a diagnostic and response strategy based on individual characteristics.

An embodiment of the present disclosure is provided to classify a person into one of multiple types related to tolerance to behavioral addiction, and to diagnose and counsel a person for behavioral addiction according to the type of the person.

The objectives to be achieved by the present disclosure are not limited to the above-mentioned objectives, and other objectives which are not mentioned will be more clearly understood by those skilled in the art from the following description and the embodiments of the present disclosure. Furthermore, it will be readily apparent that the objectives and advantages of the present disclosure can be achieved by the means and combinations thereof set forth in the claims.

### [TECHNICAL SOLUTION]

According to an embodiment of the present disclosure, a method for providing consultation for each user's type of an electronic device may be achieved by the electronic device performing a consultation step, a measurement step, a classification step, an intervention step, and a diagnosis step.

The consultation step may refer to a step of providing a voice of a digital human (AI agent) including an image in which the digital human (AI agent) is visualized and at least one query, and obtaining a user's response to the query.

In the measurement step performed after the consultation step, the electronic device may perform an operation of obtaining measurement values for five measurement items including the user's disposition, virtue, personality, cognitive faculty, and personal environments according to the user's response.

In the classification step, the electronic device may perform an operation of identifying, on the basis of the obtained measurement values, the user's type associated with tolerance to digital addiction.

In the intervention step, the electronic device may select consultation content matching the user's type, and may provide the selected consultation content in the voice of the digital human(AI agent).

The electronic device may include a first conversation model trained to generate a query and a response for the consultation step, and a plurality of second conversation models trained to provide consultation content matching each of multiple types related to tolerance to the digital addiction. In the intervention step, the electronic device may obtain the consultation content based on the second conversation model matching the user's type among the plurality of second conversation models, and may provide the obtained consultation content in the voice of the digital human((AI agent).

In the measurement step, the electronic device may obtain a measurement value for the disposition based on a user's response to a question corresponding to consistency and activity, and may set to increase a measurement value for the disposition in proportion to an increase in the positive degree of consistency and activity.

In the measurement step, the electronic device may obtain a measurement value for the virtue based on a user's response to a question corresponding to ethical standards and good faith, and may set to increase a measurement value for the virtue in proportion to an increase in the positive degree of the user's ability to practice ethical standards and good faith.

In the measurement step, the electronic device may obtain a measurement value for the personality based on a user's response to a question corresponding to catholicity and social adjustment, and may set to increase a measurement value for the personality in proportion to an increase in the positive degree of the catholicity and social adjustment.

In the measurement step, the electronic device may obtain a measurement value for the cognitive faculty based on a user's response to a question corresponding to a problem solving ability, and may set to increase a measurement value for the cognitive faculty in proportion to an increase in the positive degree of the problem solving ability, and may obtain a measurement value for the cognitive faculty based on a user's response to a question corresponding to social support or social organizational capacity, and may set to increase a measurement value for the cognitive faculty in proportion to an increase in the positive degree of the social support or social organizational capacity.

In the classification step, the electronic device may identify the user's type based on the obtained measurement values, identify the user as an avoidance type when all measurement values for the five measurement items are below a reference value, identify the user as a compromise type when the measurement values for the disposition and cognitive faculty among the five measurement items are below the reference value, and identify the user as a problem-solving type when all the measurement values for the five measurement items are above the reference value.

In the diagnosis step, the electronic device may determine a user's digital addiction degree based on exposure time to factors (e.g., digital content) causing digital addiction of the user and the user's type.

### [EFFECT OF INVENTION]

According to an embodiment of the present disclosure, it is possible to perform diagnosis and consultation for behavioral addiction (digital addiction) in response to the user's characteristics, thereby helping to establish a customized response strategy.

According to an embodiment of the present disclosure, data on the user's personality traits is collected through a query-and-response process with an artificial intelligence-based digital human((AI agent), so it is possible to identify the user's type in an environment similar to a real consultation, and it is not necessary to write a separate test, so it helps reduce fatigue and offers advantages in terms of therapeutic accessibility, efficiency, and anonymity.

Further, in a case where a client experiencing addiction has been reluctant to disclose his or her problem honestly to family members or offline counselors due to concerns about stigma, the effectiveness of treatment has often been limited. However, these limitations can be addressed by establishing a client-centered consultation model based on interactive communication with an AI-based digital human according to an embodiment of the present disclosure, thereby maximizing clinical effect.

### [BRIEF DESCRIPTION OF THE DRAWING]

FIG. 1 is a diagram illustrating the configuration of an electronic device according to an embodiment of the present disclosure.
FIG. 2 is a diagram illustrating the configuration of a user determination unit according to an embodiment of the present disclosure.
FIG. 3 is a diagram illustrating the configuration of a second conversation model according to an embodiment of the present disclosure.
FIG. 4 is a diagram illustrating a method for controlling an electronic device according to an embodiment of the present disclosure.

### [BEST MODE FOR CARRYING OUT THE INVENTION]

The above and other objectives, features, and advantages of the present disclosure will be easily understood from the following embodiments in conjunction with the accompanying drawings. However, the present disclosure may be embodied in different forms without being limited to the embodiments set forth herein. Rather, the embodiments disclosed herein are provided to make the disclosure thorough and complete and to sufficiently convey the spirit of the present disclosure to those skilled in the art, and the present disclosure is defined solely by the claims.

The terminology used herein is for the purpose of describing embodiments only and is not intended to be limiting. In the present disclosure, the singular forms are intended to include the plural forms as well, unless the context clearly indicates otherwise. The terms "comprises" and/or "comprising" when used in this specification do not preclude the presence or addition of one or more other components. The same reference numerals are used throughout the drawings to designate the same components, and the terms "and/or" specify the presence of stated components and a combination thereof. It will be understood that, although the terms "first", "second", etc. may be used herein to describe various components, these components should not be limited by these terms. These terms are only used to distinguish one component from another component. Thus, a first component could be termed a second component without departing from the teachings of the present disclosure.

Unless otherwise defined, all terms including technical and scientific terms used herein have the same meaning as commonly understood by one of ordinary skill in the art to which the present disclosure belongs. Further, terms used herein will not be interpreted in an idealized or overly formal sense unless expressly so defined herein.

The term "unit" or "module" when used in the specification means a hardware component, such as a software, FPGA, or ASIC, and the "unit" or "module" performs certain roles. However, the "unit" or "module" is not limited to software or hardware. The "unit" or "module" may be configured to be present in an addressable storage medium and may be configured to cause one or more processors to perform operations. Thus, as an example, the "unit" or "module" includes components such as software components, object-oriented software components, class components and task components, as well as processes, functions, attributes, procedures, subroutines, segments of program code, drivers, firmware, microcode, circuitry, data, databases, data structures, tables, arrays and variables. The functions provided within the components and "units" or "modules" may be combined into a smaller number of components and "units" or "modules" or further separated into additional components and "units" or "modules".

Spatially relative terms such as "below," "beneath," "lower," "above," and "upper" may be used to easily describe a correlation between one component and other components as shown in the drawings. The spatially relative terms should be understood as including different directions of components when in use or operation in addition to the directions depicted in the drawings. For example, when components depicted in the drawings are flipped, a component described as being "below" or "beneath" another component may then be positioned "above" the other component. Therefore, the exemplary term "below" may encompass both downward and upward directions. Components may also be oriented in different directions, and thus spatially relative terms may be interpreted based on such orientation.

An electronic device 10 according to an embodiment of the present disclosure may perform consultation for the purpose of prevention, diagnosis, and treatment of behavioral addiction.

Unlike substance addiction (or drug addiction), the behavioral addiction is not driven by the action of a specific substance on the brain to induce a neurotransmission adaptation stage that leads to tolerance and withdrawal, but is characterized by human behavior itself serving as the medium of addiction. Types of the behavioral addiction include digital addiction, gambling addiction, love addiction, religious addiction, power addiction, sex addiction, food addiction, shopping addiction, and study addiction.

Such behavioral addiction is known to be strongly influenced by factors such as individual behavior, psychological characteristics, and socio-environmental conditions. Thus, even with the same exposure time to the behavior, there are samples that are more vulnerable to addiction and others that are not, depending on their personal characteristics.

Due to the characteristics of such behavioral addiction, the electronic device 10 may classify users into types according to their characteristics through consultation (primary consultation) to determine the characteristics of the users, and conduct user consultation (secondary consultation) based on the user type.

Although the following description focuses on examples pertaining to digital addiction among behavioral addictions, it should be understood that the scope of preventive, diagnostic, and therapeutic operations according to the embodiment of the present disclosure is not limited to the digital addiction. Rather, it encompasses various types of behavioral addictions, including but not limited to gambling addiction.

A particular embodiment of the control operation of the electronic device 10 will be described below in detail with reference to the accompanying drawings.

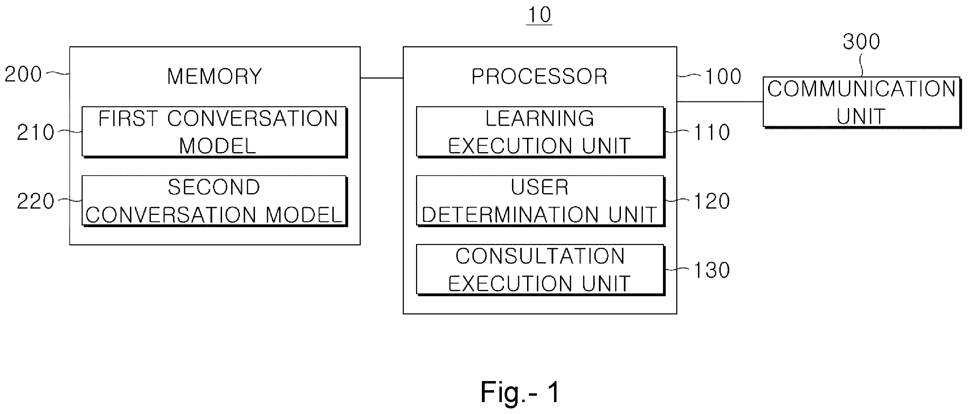

First, the configuration and function of the electronic device according to an embodiment of the present disclosure will be described with reference to FIGS. 1 to 3.

FIG. 1 is a diagram illustrating the configuration of an electronic device according to an embodiment of the present disclosure.

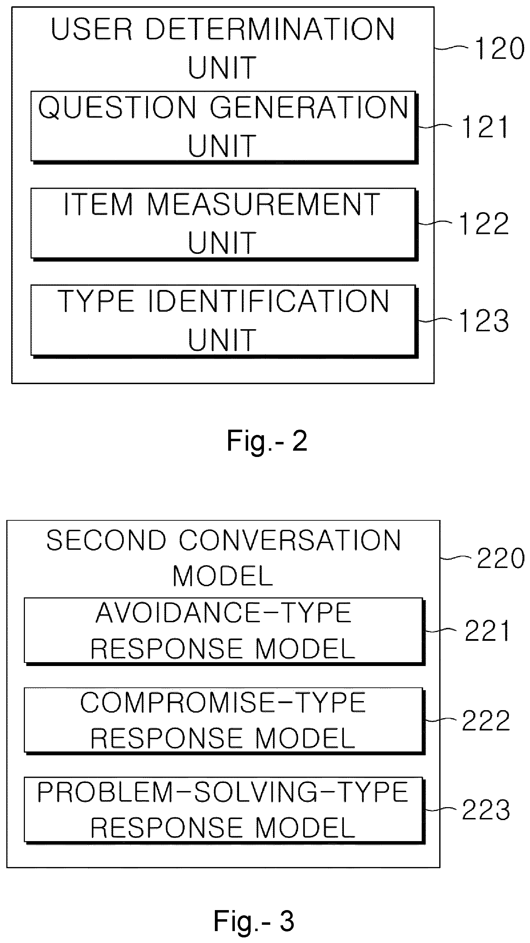

FIG. 2 is a diagram illustrating the configuration of a user determination unit according to an embodiment of the present disclosure.

FIG. 3 is a diagram illustrating the configuration of a second conversation model according to an embodiment of the present disclosure.

As shown in FIG. 1, the electronic device 10 according to an embodiment of the present disclosure may be configured to include a processor 100, a memory 200, and a communication unit 300. The processor 100 may be configured to include a learning execution unit 110, a user determination unit 120, and a consultation execution unit 130. Further, the memory 200 may be configured to include a first conversation model 210 and a second conversation model 220. Although not shown in the drawing, the memory 200 may further include an artificial intelligence model (e.g., type determination model) that performs the operation of determining the type of a user in addition to a conversation model.

As such, the electronic device 10 may be configured to include various components. Hereinafter, each component will be described in more detail.

As described above, the processor 100 of the electronic device 10 may be configured to include the learning execution unit 110, the user determination unit 120, and the consultation execution unit 130.

The learning execution unit 110 may perform the learning of an artificial intelligence model that is required to perform an operation for determining a user type based on a character evaluation theory and a consultation operation (secondary consultation) for each user type.

First, the learning execution unit 110 may train the artificial intelligence model (first conversation model) to generate the query and response content required for a primary consultation operation among operations required to determine the user type.

For example, the learning execution unit 110 may train the artificial intelligence model to perform the operation of providing a visualized image of a digital human((AI agent) and a voice of the digital human including at least one query. The digital human((AI agent) may refer to a virtual counselor who provides a consultation question to the user based on a pre-trained artificial intelligence model and responds to a user's response.

At this time, the digital human((AI agent) may provide a question to a user through a voice sound so that the user may respond and ask a question in an environment similar to talking to a real counselor.

During the process of conducting the conversation with the user, the digital human(AI agent) may provide the visualized image to the user so that the user can more easily understand the digital human's question. Further, the digital human((AI agent) may prevent the misrecognition of the user's response, and provide the user with the visualized image during the process of confirming the content of the response.

The learning execution unit 110 may train the artificial intelligence model (type determination model) that identifies the user's type based on the user's response in the primary consultation.

The learning execution unit 110 may train the artificial intelligence model (type determination model) to obtain measurement values for five measurement items (disposition, virtue, personality, cognitive faculty, and personal environments) corresponding to five elements of the character evaluation theory based on the user's response to the query, and to identify the user's type as one of three types: avoidant, compromising, and problem-solving based on the measurement value.

The learning execution unit 110 may perform the learning of the artificial intelligence model (second conversation model) that conducts consultation according to the identified user's type.

Specifically, the learning execution unit 110 may select consultation content matching the user's type, and train the artificial intelligence model (second conversation model) to provide the selected consultation content as at least one of the digital human's voice and text.

The user determination unit 120 may perform an operation to determine the user's type based on the artificial intelligence model learned by the learning execution unit 110.

The user determination unit 120 may perform the primary consultation operation to collect user response content required for determining the user's type. Further, the user determination unit 120 may classify users by types based on the user's response obtained through the primary consultation, and may classify users into one of three type (e.g., avoidant type, compromise type, problem-solving type) according to the character evaluation theory.

To explain the operation performed by the user determination unit 120 in more detail, reference will be made to FIG. 2.

As shown in FIG. 2, the user determination unit 120 may be configured to include a question generation unit 121, an item measurement unit 122, and a type identification unit 123.

The question generation unit 121 may generate a question required to conduct the primary consultation with the user by operating the first conversation model stored in the memory 200.

According to an embodiment, the question generation unit 121 may designate the initial question presented to the user to be the same regardless of who the user is. Alternatively, the question generation unit 121 may generate an initial question in response to the user's basic personal information (e.g., gender, age, occupation, etc.). Without being limited to the described method, the question generation unit 121 may generate the initial question to be presented to the user starting the primary consultation in various ways.

If a corresponding response is received from the user after generating the initial question and presenting it to the user, the question generation unit 121 may generate a follow-up question corresponding to the received user response and present it to the user.

At this time, the question generation unit 121 may generate questions to measure five items (disposition, virtue, personality, cognitive faculty, personal environments) corresponding to the character evaluation theory when generating the questions.

Specifically, a question generating method for measuring each of the five items will be described as follows.

First, the question generation unit 121 may generate a question for measuring 'disposition' among the five items. The 'disposition' is a latent variable that measures the strength and tone of one's innate temperament. The 'disposition' is an item designed to measure the consistency of a character in terms of intensity, and the degree of activity in terms of brightness or darkness. For example, in the present disclosure, the steadier, the more consistent, and the brighter the user is determined to be, a higher value may be measured for the 'disposition'.

Depending on the characteristics of the item called 'disposition', the question generation unit 121 may generate a question to evaluate consistency, such as a user's will to continue with a plan he or she has made. The question generation unit 121 may generate questions such as 'Have you ever continued a planned exercise for more than a month?" and 'Are you evaluated as a bright person by those around you?' as the question for measuring the 'disposition' item.

Next, the question generation unit 121 may generate a question for measuring the 'virtue' among the five items. The 'virtue' is a variable that measures an individual's usual ability to practice ethical standards and good faith (consistency between words and actions) established through the individual's acquired efforts. The value of 'virtue' may be measured higher as the user is determined to have ethical standards and good faith.

Depending on the characteristics of the item called ' virtue', the question generation unit 121 may generate questions such as 'Do you usually act morally, in accordance with reason and propriety, and without falsehood?', 'Do you often lack humility and place undue emphasis on appearances, even when it goes against reason or propriety?', and 'Are you lacking in integrity and consistency between your words and actions in general?'.

Next, the question generation unit 121 may generate a question for measuring the 'personality' among the five items. The 'personality' is an item designed to measure consideration and catholicity for others and social adjustment, and may be measured as having a higher value as the user is determined to have higher catholicity and social adjustment.

Depending on the characteristics of the item called 'personality', the question generation unit 121 may generate questions such as 'Are you usually very considerate and tolerant of others?', 'Are you usually lacking in consideration and catholicity for others?', 'Do you usually get along well with people around you?', and 'Do you usually have difficulty getting along with people around you?'.

Next, the question generation unit 121 may generate a question for measuring the 'cognitive faculty' among the five items. The 'cognitive faculty' is a variable that measures a person's problem-solving ability. The higher the user's problem-solving ability is evaluated, the higher the value of the 'cognitive faculty' may be measured.

Depending on the characteristics of the item called 'cognitive faculty', the question generation unit 121 may generate questions such as 'Do you tend to make good use of your own experiential knowledge and the advice of those around you?', 'Do you sometimes insist on your own experiential knowledge and problem-solving ability, leading to difficulties in the problem-solving process?', 'Do you prefer rational and fundamental problem-solving methods?', and 'Do you prefer to rely on spontaneous ideas rather than rational and fundamental problem-solving methods?'.

Finally, the question generation unit 121 may generate a question for measuring the 'personal environments' among the five items. The 'personal environments' are a variable that measures the social position and social activity of a person. Further, the 'personal environments' are a variable that measures the degree of social support from organization members or people around you, and the social achieved status and social sphere that is valued in community activities. The more a user's social support or social organizational capacity is determined to be, the higher the value of the 'personal environments' item may be measured.

Depending on the characteristics of the item called 'personal environments', the question generation unit 121 may generate questions such as 'Are you generally regarded with high expectations and respect by organization members or those around you?', 'Are you generally not regarded with high expectations and trust by organization members or those around you?', 'Are you usually proactive in helping your community and others?', and 'Are you usually not proactive in helping your community and others?'.

The question generation unit 121 determines whether to generate an additional question and whether to change the subject of the additional question in response to the degree of completion of the item measurement determined by the item measurement unit 122. If the item measurement is not completed, an additional question may be generated.

For instance, when the item measurement unit 122 determines that all of the five items has been measured, it transmits a signal to the question generation unit 121, and then the question generation unit 121 may terminate the primary consultation without generating an additional follow-up question. On the other hand, the question generation unit 121 may control to change the subject of the additional question as it receives from the item measurement unit 122 that only 4 out of 5 measurement items have been completed.

In addition, when the user's response is received, the question generation unit 121 may quantify the specificity of the response and determine the degree of specificity of an additional question based on the quantified specificity.

Methods of quantifying the specificity of the response may include, for example, the method of measuring the volume of the response, the method of measuring the diversity of words included in the response, or the method of measuring both the volume of the response and the diversity of the words.

When the obtained user response does not reach the specificity level of the response that is desired to be obtained, the question generation unit 121 may generate an additional question with a stronger degree of specificity.

For instance, if a user's response to the question, "Do you usually stick to your plans?" is below a certain standard in length (e.g., 10 characters or less) or shows a word diversity below a standard (e.g., 3 types or less), such as "yes" or "no," the question generation unit 121 may present an additional question, "Have you ever followed through with a plan for more than a month?" that enhances the level of specificity on the same subject. Likewise, in the subsequent consultation process, if the question generation unit 121 determines that the obtained response does not reach the target level of specificity, it may continuously generate an additional question with a stronger level of specificity.

The item measurement unit 122 may secure the user's response obtained by providing a question to the user through the question generation unit 121, and may measure five items (disposition, virtue, personality, cognitive faculty, and personal environments) based on the character evaluation theory based on the user's response.

The item measurement unit 122 may measure the values for the five measurement items by inputting the user's response into the artificial intelligence model (type determination model) trained by the learning execution unit 110.

Specifically, for example, the item measurement unit 122 may analyze each user's response that is input into the artificial intelligence model and record it on an n-dimensional (preferably 2-dimensional or 3-dimensional) coordinate according to a correlation value with the personality traits signified by each item of the five elements. Thereafter, the item measurement unit 122 may calculate the average of the coordinate values of points for which coordinate values are specified, and may derive a measurement value for each item among the five items by averaging the coordinate values of each calculated point. At this time, the axis of each coordinate may be designated to correspond to a sub-item of the five items (e.g., the sub-items of 'disposition' correspond to consistency and activity).

In the following, a method in which the item measurement unit 122 calculates a measurement value for each of the five items will be described. First, a process by which the item measurement unit 122 calculates the measurement value for the 'disposition' item is as follows. The item measurement unit 122 may input the user's response and calculate the correlation values for the user's tendencies and the sub-items corresponding to the ' disposition' item, namely 'consistency' and 'activity'. At this time, when the artificial intelligence model determines the correlation value between the 'disposition' item and the user's characteristics, the x-coordinate may be designed to increase in value in response to the positive degree of 'consistency' (the stronger the consistency), and the y-coordinate may be designed to increase in value in response to the degree of 'activity' (the stronger and brighter the activity).

According to various embodiments, the artificial intelligence model may be designed to record the correlation value between each item and the user characteristic in a graph that includes an additional axis after designating each axis of the graph as the sub-item of each of the five items. Further, the artificial intelligence model may be recorded by calculating a value for the reliability index of the user's response on the additional axis (e.g., the z-axis). The reliability index may be determined based on items such as the speed at which the user's responses are entered, the degree of consistency between the user's responses, etc.

The type identification unit 123 may determine the user's type as one of three preset types based on the measurement values for each of the five items measured by the item measurement unit 122.

At this time, the type identification unit 123 may classify the user into three types (e.g., avoidance type, compromise type, and problem-solving type) according to the personality evaluation theory. The avoidance type is the type with the lowest level of proactiveness and willpower to solve behavioral addiction (e.g. digital addiction) problems, while the problem-solving type is the type with the highest level of proactiveness and willpower to solve behavioral addiction problems and with strong confidence in overcoming behavioral addiction. Further, the compromise type may be an intermediate type that is more proactive in solving behavioral addiction problems than the avoidance type but less proactive than the problem-solving type. For example, a group that has a will to solve the problem of behavioral addiction but lacks confidence may be referred to as the compromise group.

Specifically, the type identification unit 123 may identify the user as the avoidance type when all the measurement values for the five measurement items are below a reference value, identify the user as the compromise type when the measurement values for the disposition and cognitive faculty among the five measurement items are below the reference value, and identify the user as the problem-solving type when all the measurement values for the five measurement items are above the reference value.

The consultation execution unit 130 of the processor 100 may perform consultation (secondary consultation) on the user's behavioral addiction in response to the user's type identified by the user determination unit 120.

The consultation execution unit 130 may select consultation content that matches the user's type, and provide the selected consultation content in the voice of the digital human(AI agent).

The consultation execution unit 130 may utilize a plurality of second conversation models trained to provide consultation content that matches each of multiple types related to tolerance to behavioral addiction (e.g., digital addiction) so as to proceed with consultation with consultation content matching the user's type.

As shown in FIG. 3, the second conversation model may be composed of three types: an avoidance-type response model 221, a compromise-type response model 222, and a problem-solving-type response model 223, corresponding to each user's type.

When the consultation execution unit 130 conducts consultation on the behavioral addiction of an avoidant user by utilizing the avoidance-type response model 221, it may set the possibility and severity of the user's behavioral addiction to the highest value among the three types and generate questions and responses necessary for consultation. Similarly, the consultation execution unit 130 may set the possibility and severity of the user's behavioral addiction to an intermediate value when it conducts consultation on the behavioral addiction of a compromise user by utilizing the compromise-type response model 222, and may set the possibility and severity of the user's behavioral addiction to the lowest value among the three types when it conducts consultation using the problem-solving-type response model 223.

By classifying the possibility and severity of behavioral addiction according to user types, the consultation execution unit 130 may assign a behavioral addiction risk level of 3 to an avoidant user, 2 to a compromise user, and 1 to a problem-solving user, respectively, for the same level of user behavior. Further, the consultation execution unit 130 may also classify the severity of behavioral addiction for the same level of user behavior into level 3, level 2, and level 1 corresponding to avoidance, compromise, and problem-solving types, and may then proceed with consultation by increasing the intensity of treatment and the degree of behavioral improvement recommendations as the level increases. Furthermore, as the degree of behavioral improvement recommendations increases, the number of encouraging sentences included during consultation to help improve addiction symptoms may also increase.

Further, according to various embodiments, the consultation execution unit 130 may generate different consultation questions and responses corresponding to user types. When users with the same level of addiction potential and severity are identified as different user types, the consultation execution unit 130 may proceed with consultation by increasing the level of treatment and the degree of behavioral improvement recommendations for users with a weaker tolerance for behavioral addiction (e.g., digital addiction). For example, avoidant users, who have the lowest tolerance for addiction, may be required to make higher levels of behavioral improvements for the same level of addiction compared to problem-solving users.

In addition, the consultation execution unit 130 may increase various other setting values (such as the digital human's voice sound volume and the consultation progress time conducted by the digital human) during the consultation process to strengthen the demand for behavioral improvement, in the case of a type with low tolerance for behavioral addiction (e.g., digital addiction).

The memory 200 may store commands and algorithms required to perform the overall operation according to an embodiment of the present disclosure. The memory 200 according to an embodiment of the present disclosure may store various artificial intelligence models necessary to identify a user type and provide consultation on the user's behavioral addiction according to the identified user type.

The memory 200 may include a first conversation model 210 trained to generate queries and responses for the consultation step when proceeding with the consultation step to determine the user's type. The memory 200 may include a second conversation model 220 trained to provide consultation content matching each user type. The second conversation model 220 may be an artificial intelligence model trained to provide consultation content matching each of multiple types related to tolerance to behavioral addiction (e.g., digital addiction).

The second conversation model 220 may be configured in multiple units to match each user type. The second conversation model 220 may be configured to include response models for each user type: avoidance type, compromise type, and problem-solving type. As illustrated in FIG. 3, the second conversation model 220 may be configured to include an avoidance-type response model 221, a compromising response model 222, and a problem-solving-type response model 223.

The communication unit 300 may be used to perform communication between the electronic device 10 of the present disclosure and a user terminal (not shown). The user terminal may be connected via the communication unit 300 to the electronic device 10 to receive user type classification and user type-specific consultation for behavioral addiction.

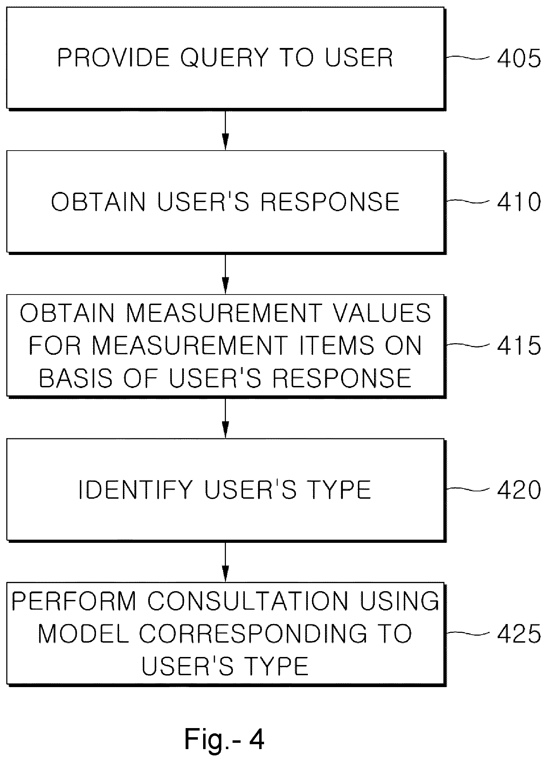

FIG. 4 is a diagram illustrating a method for controlling an electronic device according to an embodiment of the present disclosure.

As shown in FIG. 4, the electronic device 10 may perform operation 405 of providing a query to a user, operation 410 of obtaining a response from the user, operation 415 of obtaining a measurement value for a measurement item based on the response from the user, operation 420 of identifying the user type based on the measurement value, and operation 425 of performing consultation with a model corresponding to the user type.

Further, the operations 405 and 410 may correspond to the consultation step, the operation 415 of obtaining the measurement value may correspond to the measurement step, the operation 420 of identifying the user type may correspond to the classification step, and the operation 425 of performing consultation with the model corresponding to the user type may correspond to the intervention step.

Moreover, the method for controlling the electronic device 10 according to an embodiment of the present disclosure may further include a diagnostic step of determining the degree of digital addiction of the user based on the exposure time to a factor (e.g., excessive immersion in digital content, etc.) that causes behavioral addiction (digital addiction) of the user and the user type.

A method of providing consultation for each user type of an electronic device according to an embodiment of the present disclosure may be achieved by the electronic device performing a consultation step, a measurement step, a classification step, an intervention step, and a diagnosis step.

The consultation step may refer to a step in which a digital human((AI agent) provides a visualized image and at least one of voice and text of the digital human((AI agent) including at least one query and obtains a user's response to the query.

In the measurement step following the consultation step, the electronic device may perform an operation of obtaining measurement values for five measurement items including the user's disposition, virtue, personality, cognitive faculty, and personal environments according to the user's response.

In the classification step, the electronic device may perform an operation of identifying the user's type related to tolerance to the digital addiction based on the obtained measurement value.

In the intervention step, the electronic device may select consultation content matching the user's type, and provide the selected consultation content as at least one of the voice and text of the digital human((AI agent).

The electronic device may include a first conversation model trained to generate queries and responses for the consultation step and a plurality of second conversation models trained to provide consultation content matching a plurality of types related to tolerance to digital addiction. Further, the electronic device may obtain consultation content based on the second conversation model that matches the user's type among the plurality of second conversation models in the intervention step, and provide the obtained consultation content as at least one of the voice and text of the digital human((AI agent).

The electronic device may obtain a measurement value for the disposition based on the user's response to a question corresponding to consistency and activity during the measurement step, and may be set such that the measurement value for the disposition increases in proportion to the degree of consistency and activity of the user determined by the response.

The electronic device may obtain a measurement value for the virtue based on the user's response to the question corresponding to ethical standards and good faith in the measurement step, and may be set so that the measurement value for the virtue increases in proportion to the degree of the user's ability to practice the ethical standards and good faith, as determined by the response.

The electronic device may obtain a measurement value for the personality based on the user's response to a question corresponding to catholicity and social adjustment in the measurement step, and may be set so that the measurement value for the personality increases in proportion to the degree of the user's catholicity and social adjustment, as determined by the response.

In the measurement step, the electronic device may obtain a measurement value for the cognitive faculty based on the user's response to a question corresponding to the problem-solving ability, set the measurement value for the cognitive faculty to increase in proportion to the degree of the user's problem-solving ability determined by the response, obtain a measurement value for the cognitive faculty based on the user's response to a question corresponding to social support and social organizational capacity, and be set such that the measurement value for the cognitive faculty increases in proportion to the degree of the user's social support and social organizational capacity.

Further, in the classification step, the electronic device may identify the user's type based on the obtained measurement value, identify the user as the avoidance type when all the measurement values for the five measurement items are below a reference value, identify the user as the compromise type when the measurement values for the disposition and cognitive faculty among the five measurement items are below the reference value, and identify the user as the problem-solving type when all the measurement values for the five measurement items are above the reference value.

At this time, the avoidance type refers to a group with weak willpower to solve behavioral addiction problems, the compromise type refers to a group that has the will to solve behavioral addiction problems but lacks confidence, and the problem-solving type refers to a group that is confident in overcoming behavioral addiction and possesses strong willpower to solve the problems.

As described above, the electronic device may determine the user's type as one of three types based on the measurement values for the five measurement items in the classification step.

Meanwhile, according to various embodiments, the electronic device may perform an operation of classifying the type according to the user's age and gender in addition to the measurement values for the five measurement items in the classification step. For example, in the process of determining whether the electronic device is the avoidance, compromise, or problem-solving type, the electronic device may classify users into eight groups, from infants and adolescents to those in their 70s, and then apply different calculation methods for determining the type based on the attributes of each age group. In addition, the electronic device may classify users belonging to each age group into male and female, and apply different calculation methods for type determination depending on the gender attribute.

The electronic device may provide a customized service according to the user's type, after confirming the user's type.

As an example of the behavioral addiction such as digital addiction, the electronic device, in the diagnosis step, may derive data on the user's digital addiction patterns and addiction level (score) based on the user's exposure time to specific digital content and user type classification, and may set a customized intervention method according to the type of client.

The electronic device 10 according to an embodiment of the present disclosure may include a memory 200, a communication unit 300, and a processor 100.

The memory 200 may store various programs and data necessary for the operation of the electronic device. The memory 200 may be implemented as nonvolatile memory, volatile memory, flash memory, a hard disk drive (HDD), or a solid state drive (SSD).

The communication unit 300 may perform communication with an external device. In particular, the communication unit 300 may include various communication chips such as a Wi-Fi chip, a Bluetooth chip, a wireless communication chip, an NFC chip, and a low-power Bluetooth chip (BLE chip). At this time, the Wi-Fi chip, Bluetooth chip, and NFC chip communicate in the LAN mode, Wi-Fi mode, Bluetooth mode, and NFC mode, respectively. When using the Wi-Fi chip or Bluetooth chip, various pieces of connection information such as SSID and session key are first transmitted and received. After establishing a communication link using this information, various pieces of information may be sent and received. The wireless communication chip refers to a chip that performs communication according to various communication standards such as IEEE, Zigbee, 3G (3rd Generation), 3GPP (3rd Generation Partnership Project), and LTE (Long Term Evolution).

The processor 100 may control the overall operation of the user device using various programs stored in the memory 200. The processor may include RAM, ROM, a graphics processing unit, a main CPU, first to nth interfaces, and buses. At this time, the RAM, ROM, graphics processing unit, main CPU, first to nth interfaces, etc. may be connected to each other via a bus.

The RAM stores O/S and application programs. Specifically, when the electronic device boots up, the O/S may be stored in the RAM, and various application data selected by the user may be stored in the RAM.

The ROM stores a command set for system booting, etc. When a turn-on command is input and power is supplied, the main CPU copies the O/S stored in the memory 200 to the RAM according to the command stored in the ROM and executes the O/S to boot the system. When booting is complete, the main CPU copies various application programs stored in the memory 200 to the RAM and executes the application programs copied to the RAM to perform various operations.

The main CPU accesses the memory 200 and performs booting using the OS stored in the memory 200. Further, the main CPU performs various operations using various programs, contents, data, etc. stored in the memory 200.

First to n interfaces are connected to the various components described above. One of the first to n interfaces may be a network interface connected to the external device via a network.

Meanwhile, the processor may control the artificial intelligence model. In this case, it goes without saying that a control unit may include a graphics-dedicated processor (e.g., GPU) for controlling the artificial intelligence model.

The processor 100 may include one or more cores (not shown) and a graphics processing unit (not shown) and/or a connection path (e.g., a bus, etc.) for transmitting and receiving signals with other components.

The processor according to an embodiment performs a method described in connection with the present disclosure by executing one or more instructions stored in the memory 200.

Meanwhile, the processor 100 may further include RAM (Random Access Memory, not shown) and ROM (Read-Only Memory, not shown) for temporarily and/or permanently storing a signal (or data) processed within the processor. Further, the processor 130 may be implemented in the form of a system on chip (SoC), which includes at least one of a graphics processing unit, RAM, and ROM.

The memory 200 may store programs (one or more instructions) for processing and controlling the processor 100. Programs stored in the storage may be divided into multiple modules according to their functions.

The steps of the method or algorithm described in connection with an embodiment of the present disclosure may be implemented directly in hardware, in a software module executed by hardware, or through a combination thereof. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or any other form of computer readable recording medium that is well known in the art to which the present disclosure pertains.

The components of the present disclosure can be implemented as a program (or application) to be executed in combination with a computer, which is hardware, and stored in a medium. The components of the present disclosure may be implemented by software programming or software elements. Similarly, the embodiment may be implemented in a programming or scripting language, such as C, C++, Java, or assembler, including various algorithms implemented as a combination of data structures, processes, routines, or other programming constructs. The functional aspects may be implemented as algorithms executed on one or more processors 100.

Although the present disclosure has been described above with reference to preferred embodiments, it will understood by those skilled in the art that various modifications and changes may be made to the present disclosure without departing from the scope of the present disclosure. It should be noted that in order to achieve the intended effect of the present disclosure, it is not necessary to separately include all the functional blocks illustrated in the drawings or follow all the sequences illustrated in the drawings in the exact order depicted, and even if not, it may fall within the technical scope of the present disclosure described in the claims.

## Claims

1. A method for controlling an electronic device, the method comprising:
a consultation step in which the electronic device provides a voice of a digital human including an image in which the digital human((AI agent) is visualized and at least one query, and obtains a user's response to the query;
a measurement step in which the electronic device obtains measurement values for five measurement items including the user's disposition, virtue, personality, cognitive faculty, and personal environments according to the response; and
a classification step in which the electronic device identifies, on the basis of the obtained measurement values, the user's type associated with tolerance to digital addiction.

2. The method of claim 1, further comprising:
an intervention step in which the electronic device selects consultation content matching the user's type and provides the selected consultation content in the voice of the digital human(AI agent).

3. The method of claim 2, wherein the electronic device comprises:
a first conversation model trained to generate a query and a response for the consultation step; and
a plurality of second conversation models trained to provide consultation content matching each of multiple types related to tolerance to the digital addiction, and
wherein the intervention step obtains the consultation content based on the second conversation model matching the user's type among the plurality of second conversation models, and provides the obtained consultation content in the voice of the digital human.

4. The method of claim 1, wherein the measurement step obtains a measurement value for the disposition based on a user's response to a question corresponding to consistency and activity, and
obtains a measurement value for the disposition as a numerical value proportional to a degree of consistency and activity of the user.

5. The method of claim 1, wherein the measurement step obtains a measurement value for the virtue based on a user's response to a question corresponding to ethical standards and good faith, and
obtains a measurement value for the virtue as a numerical value proportional to a degree of the user's ability to practice ethical standards and good faith.

6. The method of claim 1, wherein the measurement step obtains a measurement value for the personality based on a user's response to a question corresponding to catholicity and social adjustment, and
obtains a measurement value for the personality as a numerical value proportional to a degree of the user's catholicity and social adjustment.

7. The method of claim 1, wherein the measurement step obtains a measurement value for the cognitive faculty based on a user's response to a question corresponding to a problem solving ability, and
obtains a measurement value for the cognitive faculty as a numerical value proportional to a degree of the user's problem solving ability.

8. The method of claim 1, wherein the measurement step obtains a measurement value for the personal environments based on a user's response to a question corresponding to social support or social organizational capacity, and
obtains a measurement value for the personal environments as a numerical value proportional to a degree of the user's social support or social organizational capacity.

9. The method of claim 1, wherein the classification step identifies the user's type based on measurement values for five measurement items of the disposition, virtue, personality, cognitive faculty, and personal environments,
identifies the user as an avoidance type, which is defined as a group with weak willpower to solve a problem, when all measurement values for the five measurement items are below a reference value,
identifies the user as a compromise type, which is defined as a group with willpower to solve a problem but low confidence, when the measurement values for the disposition and cognitive faculty among the five measurement items are below the reference value, and
identifies the user as a problem-solving type, which is defined as a group with strong willpower and confidence in solving a problem, when all the measurement values for the five measurement items are above the reference value.

10. The method of claim 1, further comprising:
a diagnosis step of determining a user's digital addiction degree based on exposure time to factors causing digital addiction of the user and the user's type.

11. An electronic device, comprising:
a memory;
a communication unit communicating with at least one user terminal; and
a processor providing a voice of a digital human(AI agent) including an image in which the digital human is visualized and at least one query, through the communication unit, obtaining a user's response to the query, obtaining measurement values for five measurement items including the user's disposition, virtue, personality, cognitive faculty, and personal environments according to the response, and identifying, on the basis of the obtained measurement values, the user's type associated with tolerance to digital addiction.

12. A non-transitory computer-readable medium storing at least one instruction that is executed by a processor of an electronic device to cause the electronic device to perform the control method of claim 1.
